(19) Europäisches Patentamt

European Patent Office

Office européen des brevets

(11) Veröffentlichungsnummer : **0 495 896 B1**

(12) **EUROPÄISCHE PATENTSCHRIFT**

(45) Veröffentlichungstag der Patentschrift :
08.06.94 Patentblatt 94/23

(51) Int. Cl.⁵ : **A61K 39/21**

(21) Anmeldenummer : **90915992.3**

(22) Anmeldetag : **12.10.90**

(86) Internationale Anmeldenummer :
**PCT/EP90/01729**

(87) Internationale Veröffentlichungsnummer :
**WO 91/05567 02.05.91 Gazette 91/10**

(54) **VAKZINE ZUM SCHUTZ VOR HIV-VIRUSINFEKTIONEN, VERFAHREN ZU IHRER HERSTELLUNG UND IHRE VERWENDUNG ALS DIAGNOSTIKUM UND IMMUNTHERAPEUTIKUM.**

(30) Priorität : **14.10.89 DE 3934366**

(43) Veröffentlichungstag der Anmeldung :
**29.07.92 Patentblatt 92/31**

(45) Bekanntmachung des Hinweises auf die
Patenterteilung :
**08.06.94 Patentblatt 94/23**

(84) Benannte Vertragsstaaten :
**AT BE CH DE DK ES FR GB GR IT LI LU NL SE**

(56) Entgegenhaltungen :
**EP-A- 0 283 327
EP-A- 0 347 365
PROCEEDINGS OF THE NATL. ACADEMY OF
SCIENCES USA, Band 85, August 1988; J.F.
ZAGURY et al., Seiten 5941-5942
NATURE, Band 339, Nr. 6223, 01 Juni 1989;
London (GB); V.M. HIRSCH et al., Seiten
389-392
PROCEEDINGS OF THE NATL. ACADEMY OF
SCIENCES USA, Band 86, April 1989; H. KU-
HNEL et al., Seiten 2382-2387**

(73) Patentinhaber : **CHEMOTHERAPEUTISCHES
FORSCHUNGSINSTITUT
GEORG-SPEYER-HÄUS ZU FRANKFURT A.M.
Paul-Ehrlich-Strasse 42-44
D-60596 Frankfurt (DE)**

(72) Erfinder : **DIETRICH, Ursula
Gehspitz 6
D-6236 Eschborn (DE)**
Erfinder : **ADAMSKI, Michalina
Bickenbacher Weg 22
D-6000 Frankfurt 71 (DE)**
Erfinder : **VON BRIESEN, Hagen
Ringstrasse 31
D-6242 Kronberg (DE)**
Erfinder : **KÜHNEL, Herbert
Mainstrasse 7E
D-6073 Egelsbach (DE)**
Erfinder : **RÜBSAMEN-WAIGMANN, Helga
Königsteinerstrasse 113
D-6232 Bad Soden (DE)**

(74) Vertreter : **von Kreisler, Alek, Dipl.-Chem. et al
Patentanwälte
von Kreisler-Selting-Werner
Postfach 10 22 41
D-50462 Köln (DE)**

## Beschreibung

Gegenstand der Erfindung sind Vakzine zum Schutz vor HIV-Virusinfektionen, ein Verfahren zu ihrer Herstellung sowie ihre Verwendung in einem Tiermodell zur Diagnose von AIDS und zur Differenzierung von pathogenen und apathogenen HIV-Infektionen.

Bisherige antivirale Arzneimittel, denen auch eine Wirkung gegen HIV-Viren zugesprochen wird, haben den Nachteil, daß sie ein meist geringes antivirales Wirkungsspektrum besitzen, welches mit einer relativ hohen Toxizität verbunden ist. Viele dieser Substanzen wirken gegen eine virale Thymidinkinase oder eine virale Polymerase. Jedoch ist bereits während der Therapie mit vielen dieser Substanzen eine Resistenzbildung der infizierenden Viren beobachtet worden.

Zur Gruppe der Substanzen, bei denen eine Wirkungen gegen HIV-Viren festgestellt wurde, gehört Foscarnet (Phosphonoformiat). Diese Substanz wirkt als Hemmstoff der viralen reversen Transkriptase, ist jedoch wegen seiner in klinischen Versuchen ermittelten Toxizität nicht für die Prophylaxe oder Therapie von Retrovirus-Infektionen geeignet (B. Öberg, "Antiviral effects of phosphonoformiate (PFA, Foscarnet Sodium)", Pharm. Ther. 19 (1983) p. 387 - 415.

Ein weiterer Hemmstoff der reversen Transkriptase ist die Substanz Suramin. Sie ist jedoch aufgrund ihrer Toxizität für den Säugetierorganismus ebenfalls nicht für eine Prophylaxe oder Therapie von HIV-Virusinfektionen geeignet (H. Mitsuya et al. "Suramin Protection of T-Cells in vitro against Infectivity and Cytopathic Effect of HTLV-III", Science 226 (1984), p. 172 - 174).

Die weitere Entwicklung führte dann zu Hemmstoffen der reversen Transkriptase, die weniger toxisch auf den Säugetier-Organismus sind. Dazu gehören Stoffe wie Dextransulfat und Pentosanpolysulfat, die nachweislich in vivo einen inhibierenden Effekt auf HIV I-Viren zeigen. Dies wird in den Offenlegungsschriften DE 3 601 136 und EP 0 293 826 beschrieben.

Bisherige Bemühungen, Mittel zur Prophylaxe und Therapie von HIV-Infektionen zu entwickeln, konzentrieren sich daher auf eine Hemmung der reversen Transkriptase des HIV I-Virus.

Neben der chemotherapeutischen Behandlung von HIV-Infektionen besteht prinzipiell die Möglichkeit der Gen- oder Immuntherapie. Die Gentherapie umfaßt das Einschleusen von Teilen viraler Nukleinsäuren in menschliche Zellen, insbesondere in die Zielzellen des HIV-Virus, beispielsweise die CD4 positiven Zellen des Immunsystems. Durch Bildung einer "antisense" RNA, d.h. einer zur messenger RNA (m-RNA) komplementären RNA, kann dann die virale m-RNA neutralisiert werden und somit die Weitervermehrung des Virus unterbunden werden. In einer anderen Form können auch direkt Oligonukleotide oder mit ihnen chemisch verwandte Strukturen, die zur m-RNA komplementär sind, eingesetzt werden. Bei der Immuntherapie werden nach der Infektion Antigene gegeben, von denen eine Unterstützung der Immunantwort gegen HIV erwartet wird.

Um eine weitere Ausbreitung der AIDS Epidemie zu verhindern, wäre es jedoch dringend erforderlich, eine Vakzine zum Schutz vor HIV-Infektionen zu entwickeln. Das Hauptproblem hierbei ist die hohe Variabilität der HIV-Viren: eine Schutzimpfung soll und muß alle möglichen Virusvarianten erfassen. Ein Weg dazu ist, durch Vergleich möglichst vieler und evolutionär weit entfernter HIV-Virusvarianten, konservierte Bereiche in den viralen Antigenen festzustellen, die dann als Peptide im Menschen die Bildung von protektiven Antikörpern hervorrufen können. Alternativ können die Peptide in einem anderen Organismus zur Bildung von Antikörpern eingesetzt werden, so daß die Antikörper direkt dem Menschen als Schutz gegeben werden. Neben konservierten Peptiden muß eine wirkungsvolle Vakzine aber auch solche Peptide erfassen, die von sehr divergenten Stämmen abstammen.

Zwei Virustypen, HIV-1 und HIV-2, konnten aus Patienten mit der Immunschwächekrankheit AIDS isoliert werden (Barre-Sinoussi et al., Science 220, 868-871; Clavel et al., Science 233, 343-346, 1986). Beide sind Retroviren aus der Unterfamilie der Lentiviren mit Tropismus für CD4-positive Zellen. HIV-2 unterscheidet sich von HIV-1 in mehrfacher Hinsicht: erstens unterscheiden sich die Antigene in ihrer Größe und in ihren Epitopen. Das ist der Grund dafür, daß HIV-2 Viren nur sehr schwach von serologischen Tests für HIV-1 erkannt werden. Zweitens ist auch die genetische Struktur von HIV-2 etwas anders, indem HIV-1 das Gen vpu und HIV-2 das Gen vpx besitzt. Drittens unterscheiden sich beide Virustypen in ihren Nukleotidsequenzen; die Homologie zwischen HIV-1 und HIV-2 beträgt nur 55-60 %, während die Homologie zwischen unabhängig isolierten HIV-1 Stämmen 87-94 %, die zwischen verschiedenen HIV-2 Stämmen 87-90 % beträgt. Viertens ist auch das Hauptverbreitungsgebiet beider Virustypen in Afrika verschieden: HIV-1 ist in Zentralafrika endemisch, während HIV-2 hauptsächlich in Westafrika vorkommt.

HIV-1 und HIV-2 sind bestimmten Immundefizienzviren der Affen (SIV) näher verwandt als untereinander. Der nächste Verwandte von HIV-1 ist das Virus aus dem Schimpansen, $SIV_{cpz}$ (Huet et al., Nature 345, 356-359, 1990) mit etwa 75 % Homologie, während die Viren aus Makaken, $SIV_{mac}$ (Franchini et al., Nature 328, 539-543, 1987), und aus Mangaben, $SIV_{sm}$ (Hirsch et al., Nature 339, 389-392) mit jeweils etwa 75 % Homologie dem menschlichen HIV-2 am ähnlichsten sind. HIV-2, $SIV_{mac}$ und $SIV_{sm}$ sind vom gleichen Serotyp und sind daher als unterschiedliche Subtypen einer großen Virusgruppe anzusehen.

Proc. Natl. Acad. Sci., USA, Vol. 86, pp. 2383 - 2387, April 1989 beschreibt die Isolierung von HIV-2-Viren (HIV-$2_{D194}$ und HIV-$2_{D205}$). Weiterhin wird erwähnt, daß HIV-$2_{D205}$ gegenüber bisher gefundenen HIV-2-Viren hochdivergent ist und genetisch gleichweit von den Affenviren (SIV) entfernt ist wie von anderen HIV-2-Stämmen.

Die EP-A-0 283 327 beschreibt Peptide, die immunologische Eigenschaften bezüglich HIV-2 besitzen. Es werden die Hüllprotein bestimmenden Sequenzen env-6 und env-8 beschrieben, diese sind jedoch nicht von HIV-$2_{D205}$ stammend und weisen andere Amminosäuresequenzen auf.

In der älteren, aber nicht vorveröffentlichten EP 89 710 057.4 (= EP-A-0 347 365) wird eine HIV-2-Virusvariante, nämlich HIV-$2_{D205}$ (auch $HIV_{ALT}$ genannt) beschrieben, die aus dem entsprechenden Virusisolat HIV-$2_{D205}$ kloniert werden kann. Das Virusisolat HIV-$2_{D205}$ ist gemäß Budapester Vertrag unter der Nummer ECACC V 87 122 304 am 23.12.1987 bei der European Collection of Animal Cell Cultures (ECACC), Porton Down, Salisbury, Wiltshire, Großbritannien SP40JG hinterlegt worden. Ebenfalls beschrieben werden die RNA und die davon abgeleiteten DNAs sowie die Proteine der Virusisolate.

Das Virus HIV-$2_{D205}$ definiert einen alternativen Subtyp der HIV-2/$SIV_{mac}$/$SIV_{sm}$ Virusgruppe. Dieses Virus stammt aus einer asymptomatischen HIV-positiven Ghanesin. HIV-$2_{D205}$ reagierte stark im HIV-2 Test, viele viralen Antigene hatten jedoch unterschiedliche Größe. Die Bestimmung der Nukleotidsequenz ergab jedoch, daß es sich bei HIV-$2_{D205}$ um einen hoch divergenten Stamm handelt. Die Homologie zwischen HIV-$2_{D205}$ und den bereits sequenzierten HIV-2 Stämmen (ROD: Guyader et al., Nature 326, 662-669, 1987; NIHZ: Zagury et al., PNAS 85, 5941-5945, 1988; ISY: Franchini et al., PNAS 86, 2433-2437, 1989; D194: Kühnel et al., PNAS 86, 2383-2387, 1989) beträgt nur etwa 76 %. HIV-$2_{D205}$ steht also genetisch genau zwischen diesen HIV-2 Stämmen (hier als ROD-Typ bezeichnet) und den Affenviren $SIV_{mac}$/$SIV_{sm}$ (jeweils etwa 75 % Homologie). Stammbaumanalysen ergaben, daß der Ursprung von HIV-$2_{D205}$ noch vor der Trennung zwischen den HIV-2 ROD-Typ Viren und den Affenviren $SIV_{mac}$/$SIV_{sm}$ anzusiedeln ist. HIV-$2_{D2O5}$ steht also evolutionär einem gemeinsamen Vorfahren der gesamten Virusgruppe HIV-2/$SIV_{mac}$/$SIV_{sm}$ sehr nähe.

Es ist unbedingt erforderlich, daß Viren wie HIV-$2_{D205}$ bei der Entwicklung von Vakzinen, Therapeutika oder Diagnostika berücksichtigt werden. Vakzine müssen gegen alle Varianten des infektiösen Erregers schützen. Leiten sich die Vakzine von konstanten Regionen eines phylogenetisch alten Virus ab, so ist zu erwarten, daß sie ein sehr breites Sprektrum an Varianten erfassen. Das gleiche gilt für Peptide, Antikörper, Nukleinsäuren oder weitere aus den Nukleotidsequenzen abgeleitete Substanzen, die als Gen- oder Immuntherapeutika gegen HIV eingesetzt werden. Diagnostika, die zwischen Viren des ROD-Typs und des D205-Typs unterscheiden sollen, müssen jedoch von denjenigen Sequenzbereichen abgeleitet werden, die zu möglichst unterschiedlichen Antigenen führen.

Zusätzlich bietet sich ein Virus wie HIV-$2_{D205}$, das phylogenetisch zwischen den HIV-2 und den Affenviren $SIV_{mac}$/$SIV_{sm}$ steht, zur Etablierung eines Affenmodells für AIDS an. HIV-$2_{D205}$ ist bei weitem das diesen Affenviren ähnlichste Immundefizienzvirus, das aus einem Menschen isoliert wurde und könnte sich daher in Affen ähnlich wie ein Affenvirus verhalten und möglicherweise auch Krankheit induzieren.

Bei der bisherigen Entwicklung einer Vakzine gegen HIV-Virusinfektionen ergibt sich die Schwierigkeit, daß es zur Zeit kein geeignetes Tiermodell für die Krankheit gibt. Deshalb kommt die Entwicklung von Impfstrategien, beispielsweise gegen die Eigenschaft des Virus, die Zusammensetzung seiner proteinhülle zu verändern und seine eigenen Gene in das Erbmaterial des Wirtes einzubauen, nur langsam voran. Weiterhin muß die Vakzine einen ausreichenden Schutz gegen die vollständige Palette der bisher gefundenen zahlreichen HIV-Varianten gewährleisten. Der Impfstoff selbst darf aber auch nicht das Risiko einer HIV-Infektion bergen.

Bei der weiteren Sequenzierung des HIV-$2_{D205}$-Virus und anschließend durchgeführten Stammbaumanalysen wurde jetzt überraschenderweise gefunden, daß HIV-$2_{D205}$ genetisch gleich weit von den bisher beschriebenen HIV-2-Stämmen und den Affenviren $SIV_{mac}$ und $SIV_{sm}$ entfernt ist. HIV-$2_{D205}$ steht somit einem gemeinsamen Vorläufer der HIV-2/$SIV_{mac}$/$SIV_{sm}$-Gruppe evolutionär sehr nahe und ist somit ein sehr altes Virus.

Daher bietet sich dieses Virus als Ausgangsstoff zur Herstellung einer Vakzine, eines Gen- oder Immuntherapeutikums mit breitem Schutzspektrum gegen die Viren dieser Gruppe an. Gleichzeitig könnte sich HIV-$2_{D205}$ aufgrund seiner engen Verwandtschaft zu den Affenviren besonders gut zur Etablierung eines Tiermodells eignen. Für die Etablierung eines Tiermodells wird HIV-$2_{D205}$ auf geeignete Primaten (z. B. Rhesusaffen) übertragen und die Etablierung der Infektion und gegebenenfalls das Auftreten von Symptomen beobachtet. Die Etablierung eines Tiermodells mit HIV-$2_{D205}$ als Virus erlaubt zugleich die Testung der hergestellten Vakzine, Gentherapeutika oder Immuntherapeutika.

Aufgabe der vorliegenden Erfindung ist es daher, eine Vakzine zum Schutz vor HIV-Virusinfektionen zur Verfügung zu stellen, die ein möglichst breites Wirkungsspektrum gegen verschiedene Typen der HIV-2/SIV-Gruppe besitzt und die Etablierung eines zur Entwicklung des Impfstoffs nötigen Tiermodells ermöglicht.

Diese Aufgabe wird dadurch gelöst, daß die Vakzine aus dem Virus HIV-2$_{D205}$ mindestens eines der Peptide

1. LFETSIKPCVKL      2. ESCDKHYWD

3. RFRYCAPPG      4. LLRCNDTNYSGF

5. STWFGFNGTRAENRYIYWH      6. DNRTIISLN

7. NELDRFGLAESLLE      8. PLVPTGSENLKSL

9. PLSPRTLNAWVKL

10. EEKKFGAEVVPGFQALSEGCTPYEINQMLNCV

11. GLQKCVRMYNPTNILD      12. FQSYVDRFYKSLRAEQTD

13. QNANPDCKLVLKGL      14. NPTLEEMLTACQG

15. GGPGQKARLMAEALKE      16. ARQCRAPRRQGCWKCGK

oder Kombinationen derselben enthält.

Eine bevorzugte Ausführungsform ist eine Vakzine, die zum therapeutischen Einsatz vor der Infektion geeignet ist. Eine weitere bevorzugte Ausführungsform ist, daß die Vakzine als Immuntherapeutikum zum Einsatz nach der Infektion geeignet ist und ebenfalls ein breites Variantenspektrum abdeckt.

Weiterhin ist auch ein Verfahren zur Herstellung einer Vakzine zum Schutz vor HIV-Virusinfektionen aus dem Virus HIV-2$_{D205}$ sowie die Verwendung des Virus HIV-2$_{D205}$ in einem Tiermodell zur Diagnose von AIDS Gegenstand der Erfindung.

In einer weiteren Ausführungsform wird das Virus in ein geeignetes Wirtstier übertragen und dort vermehrt. Die gebildeten Antikörper werden sodann nach entsprechender Aufarbeitung, wie dies auch im Stand der Technik für andere Impfstoffe bekannt ist, als Passiv-Impfstoff gegen HIV-Virusinfektionen verwendet. Als Wirtstiere können insbesondere geeignete Affenarten verwendet werden, aber auch Tiere, die immunisiert werden können, ohne krank zu werden (z.B. Kaninchen) (Filice et al., Nature 335, 366 - 369, 1988).

Für die Differentialdiagnostik werden ausgewählte Bereiche der DNA von HIV-2$_{D205}$ herangezogen, die bei dem Prototyp HIV-2$_{ROD}$ entweder gar nicht vorkommen oder erheblich abweichen (mehr als 30 %). Von diesen Bereichen werden Peptide oder Nukleinsäuren für die Diagnostik nach den üblichen Methoden hergestellt (Markierung mit radioaktiven Isotopen, Immunfluoreszenztest, ELISA, etc.). Ein Bereich, der bei HIV-2$_{D205}$ einzigartig ist, ist z. B. eine 54 Basenpaare (bp) Insertion im überlappungsbereich der beiden offenen Leserahmen gag und pol mit der Sequenz

AACCCAGCAGAGGGCATGACACCTCGGGGGGCGACACCATCTGCGCCCCCTGCA.

Andere sehr variable Bereiche sind neben dem Hüllprotein env die Gene rev und vif.

Bisherige Untersuchungen des HIV-2$_{D205}$-Virus, einem Isolat einer asymptomatischen Ghanesin, zeigten Wachstum auf Lymphocyten ohne zytopathische Effekte und ein gutes Wachstum auf Makrophagen (Kühnel, H. et al., Proc. Natl. Acad. Sci., U.S.A. 86, p. 2383-2387 (1989)).

Bei der weiteren Untersuchung des HIV-2$_{D205}$-Virus wurde gefunden, daß der Klon D 205.7 7817 Basenpaare (bp) eines proviralen Genoms ausgehend von dem linken "long terminal repeat" (LTR) enthält. Der Klon besitzt folgende virale Gene vollständig: "gag" für die Kernproteine des Virus, "pol" für die Integration und Replikation (Protease, reverse Transkriptase, Integrase), den Infektivitätsfaktor "vif", das für HIV-2 und die Affenviren SIV$_{mac}$ und SIV$_{sm}$ typische Gen "vpx", das für schnelles Wachstum verantwortliche Gen "vpr" und die beiden ersten Exons der positiven Regulatorgene "tat" und "rev". Der für das äußere Hüllprotein kodierende Bereich ist zu 4/5 im Klon enthalten, die Sequenz des negativ regulierenden Faktors "nef" zur Hälfte insofern als er mit der rechten LTR überlappt und die linke und rechte LTR identisch sind.

Die Sequenz wurde verglichen mit den bisher bekannten HIV und SIV Sequenzen (siehe Tabelle 1). Überraschenderweise wurde gefunden, daß die Nukleotidhomologie des gesamten Klons HIV-2$_{D205}$ zu den bereits bekannten HIV-2-Sequenzen nur 76,2 - 76,8 % beträgt, während die Homologie der anderen HIV-2 Sequenzen untereinander mit 87,0 - 89,3 % weit größer ist. Die Homologie zu SIV$_{sm}$ und SIV$_{mac}$ beträgt 76,4 bzw. 75,0 %, d.h. HIV-2$_{D205}$ ist gleich weit von diesen beiden Affenvirus-Sequenzen entfernt wie von HIV-2. Die Homologie zu SIV$_{AGM}$ und HIV-1 liegt im gleichen Rahmen wie für die übrigen HIV-2 Isolate.

Sequenzvergleiche einzelner genetischer Abschnitte von HIV-2$_{D205}$, HIV-2$_{ROD}$, SIV$_{sm}$ und SIV$_{mac}$ zeigten, daß die Variabilität im Nukleotid-Bereich von 4,6 % (für R) bis zu 35,9 % (für rev exon 1) und im Aminosäure-Bereich 12,8 % (für gag) bis zu 47,6 % (für rev exon 1) reicht (siehe Tabelle 2).

Trotz dieser Variabilität waren funktionell relevante Sequenzen oder proteincharakteristika wie Hydrophilie oder Ladung in hohem Grade erhalten.

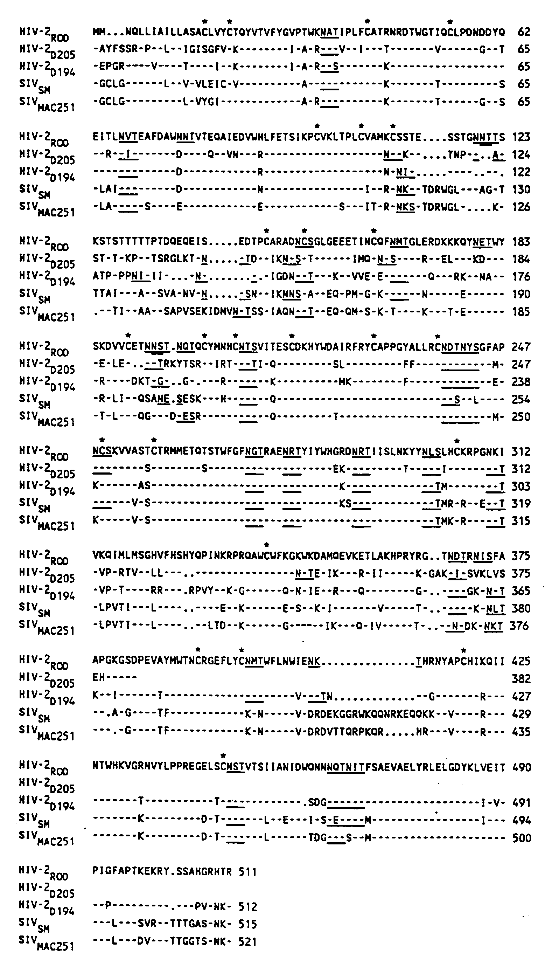

Der sequenzierte Teil des externen Glycoproteins (gp) des HIV-2$_{D205}$ unterschied sich zwar um 33 % von dem des HIV-2$_{ROD}$, hatte aber dasselbe Muster von konservierten und variablen Bereichen wie die anderen HIV-2 und SIV$_{sm}$/SIV$_{mac}$ Sequenzen (siehe Figur 2). Alle Cystein-Reste, die in den externen Glycoproteinen des HIV-2$_{ROD}$ gefunden wurden, waren erhalten. Abweichungen in den externen Glycoproteinen beruhen auf dem Austausch einzelner Aminosäuren und geringfügiger Deletionen oder Insertionen meist in den variablen Regionen. Zusätzlich wurde eine große Anzahl veränderter Glycosylierungsstellen beobachtet. Bei dem externen gp von HIV-2$_{D205}$ sind lediglich 13 von 21 potentiellen N-Glycosylierungsstellen des entsprechenden gp von HIV-2$_{ROD}$ enthalten, was die Bedeutung der Glycosylierung bei der Erzeugung der Hüllenvariabilität unterstreicht.

Durch Immunpräzipitation viraler Proteine aus infizierten Zellen und anschließender Analyse mit Hilfe der SDS-Polyacrylamid Gelelektrophorese wurde gefunden, daß die äußeren Hüllproteine des HIV-2$_{D205}$-Virus größere Molekulargewichte besitzen als die des HIV-2$_{ROD}$-Typs.

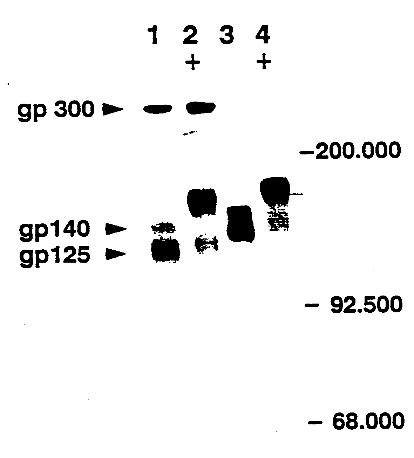

Der Vorläufer des Hüllproteins bildet bei HIV-2$_{D205}$ im Gegensatz zu den anderen HIV-2 und SIV$_{mac}$/SIV$_{sm}$ Stämmen kein Dimer (siehe Figur 3).

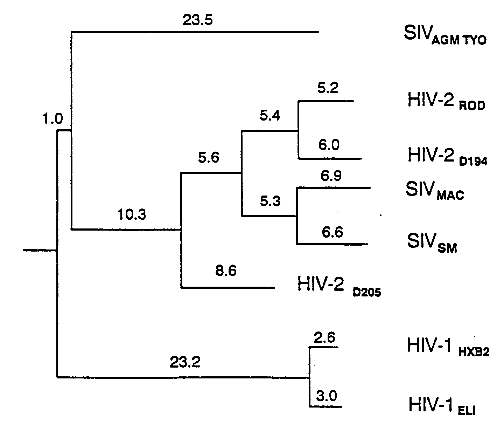

Die Dimerbildung, die für eine korrekte Prozessierung der Hüllproteinvorläufer dieser Virusgruppe, im Gegensatz zu den HIV-1 Viren, notwendig sein soll, ist also entweder eine relativ neue Entwicklung innerhalb der HIV-2/SIV$_{mac}$/ SIV$_{sm}$ Gruppe oder HIV-2$_{D205}$ stellt einen eigenständigen Virustyp dar. Für den letzteren Punkt spricht die Einordnung von HIV-2$_{D205}$ innerhalb des phylogenetischen Stammbaums der HIV/SIV Viren (Figur 4). Der Stammbaum basiert auf der Nukleotidvariation im 3'-Teil des HIV-2$_{D205}$. Darin ist zu erkennen, daß sich HIV-2$_{D205}$ um 24,8 % vom HIV-2 Prototyp HIV-2$_{ROD}$ unterscheidet und um 26,1 % bzw. 26,4 % von SIV$_{sm}$ und SIV$_{mac}$. Dieser phylogenetische Abstand stimmt gut überein mit den durch Sequenzvergleiche direkt bestimmten Abweichungen in der Nukleotidsequenz (Tabelle 1). Nach der Stammbaumanalyse wird das HIV-2$_{D205}$ Virus einem gemeinsamen Vorläufer der Typen HIV-2/SIV$_{sm}$/ SIV$_{mac}$ mit einer Divergenz von 8,6 % zugeordnet. Es wird daher die Bezeichnung HIV-2$_{ALT}$ für HIV-2$_{D205}$ vorgeschlagen.

Es ist dem Fachmann bekannt, daß die Pathogenität eines Virus mit zunehmender Adaptationszeit an seinen Wirt abnimmt. So ist zum Beispiel das Virus SIV$_{sm}$ aus sooty mangabey nicht pathogen für diese Affenart, wird es aber in eine andere Affenart neu eingebracht, z. B. in die Makaken, so wird es für diese Art pathogen. HIV-2$_{D205}$ stammt aus einer asymptomatischen Person, macht keinen zytopathischen Effekt auf Lymphocyten und ist ein relativ altes Virus. Es könnte sich also bei diesem Virus um einen nicht pathogenen Subtyp aus der HIV-2 Gruppe handeln. Es ist deshalb ebenso Bestandteil dieser Erfindung, HIV-2$_{D205}$ oder Antigene, Peptide oder Nukleinsäuren davon, für die differentielle Diagnostik zur Unterscheidung zwischen Infektionen des Typs HIV-2$_{D205}$ und solchen des Prototyps HIV-2$_{ROD}$ einzusetzen. Das Virus HIV-2$_{D205}$ eignet sich daher auch zur Herstellung von Gen-, Immuntherapeutika und Diagnostika.

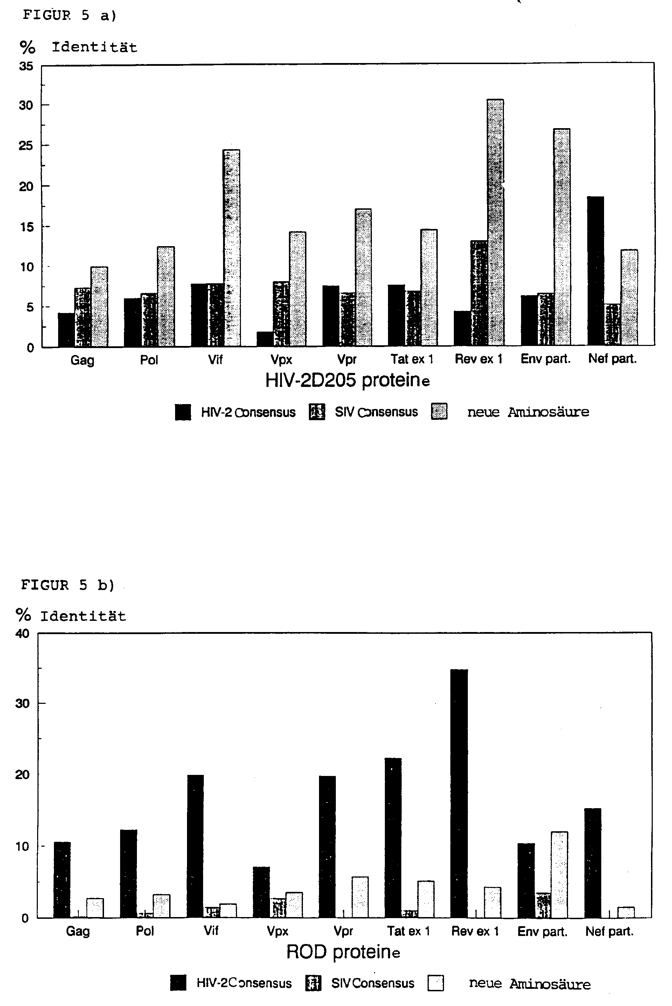

Durch Vergleichen der Aminosäuresequenzen der Proteine von HIV-2$_{D205}$ mit den Aminosäure-Consensus-Sequenzen, die für die einzelnen Gene aus allen bereits veröffentlichten HIV-2 und SIV$_{mac}$/SIV$_{sm}$ Sequenzen abgeleitet wurden, ergeben sich für den vorhandenen env-Bereich 6 konservierte Bereiche mit mindestens 9 100 %ig konservierten Aminosäuren, für gag 11 Bereiche mit mindestens 13 100 %ig konservierten Aminosäuren (Figur 5). Peptide aus diesen Bereichen bieten sich vorzugsweise für eine Vakzine mit breitem Wirkungsspektrum an, besonders, wenn die Vakzine noch aus einer Kombination dieser Peptide besteht. Es handelt sich dabei um folgende Peptide:

1.  LFETSIKPCVKL                    2.  ESCDKHYWD

3.  RFRYCAPPG                       4.  LLRCNDTNYSGF

5.  STWFGFNGTRAENRYIYWH             6.  DNRTIISLN

7.  NELDRFGLAESLLE                  8.  PLVPTGSENLKSL

9.  PLSPRTLNAWVKL

10. EEKKFGAEVVPGFQALSEGCTPYEINQMLNCV

11. GLQKCVRMYNPTNILD                12. FQSYVDRFYKSLRAEQTD

13. QNANPDCKLVLKGL                  14. NPTLEEMLTACQG

15. GGPGQKARLMAEALKE                16. ARQCRAPRRQGCWKCGK

Diese Peptide werden synthetisch hergestellt und wenn vorteilhaft chemisch modifiziert. Diese Peptide sowie kürzere bis zu 7 Aminosäuren lange Unterpeptide davon sind ebenso Bestandteil dieser Erfindung wie ihre modifizierten Formen. Weiterhin sind auch Peptide aus konstanten Bereichen anderer Gene von HIV-2$_{D205}$ Bestandteil dieser Erfindung, sofern die Abweichung von der Consensus-Sequenz nicht mehr als 20 % beträgt.

Ebenso werden die sehr abweichenden Proteinbereiche (wie z. B. rev (aa 2-11), vif (aa 186-202), env (aa 2-23;, 118-204;, etc.) für die Vakzine-Herstellung als geeignet angesehen, da bei ihnen die Induktion von Mechanismen angenommen wird, die die natürliche (und erfolgreiche) Abwehr gegen HIV-2$_{D205}$ bedingen. Diese können ebenfalls in der erfindungsgemäßen Vakzine enthalten sein.

Die gleichen Bereiche wie für die Vakzine gelten auch für die Gen- und Immuntherapie. Von konstanten Bereichen erhofft man sich ein möglichst breites Spektrum an Virusvarianten, das durch die Therapeutika erfaßt wird. Die weniger konstanten Bereiche könnten gerade die speziellen Immunantworten verursachen, die die Viren in Schach halten.

Für die Differentialdiagnostik kommen Peptide oder Nukleinsäuren in Frage, die möglichst unterschiedlich von dem Prototyp HIV-2$_{ROD}$ sind. Solche sind neben der einzigartigen 54 bp Insertion (s.o.) im gag/pol Überlappungsbereich besonders in den Genen rev und vif zu finden.

Durch die verwandtschaftliche Nähe zu den Affenviren und ausgehend von dem Grundsatz, daß Vakzine aus Vorstämmen eine breitere Schutzwirkung entfalten können ist HIV-2$_{D205}$ besonders geeignet zur Herstellung von Vakzinen zum Schutz vor HIV-Infektionen. Dieser Virus kann im Affen als geeignetem Wirt vermehrt werden, und die entstandenen Antikörper können als Vakzine für eine Anwendung beim Menschen Verwendung finden. Die Vakzine schützt damit vor der Infektion eines möglichst breiten Spektrums von Virusvarianten.

Weiterhin ist es möglich Peptidteste oder PCR-Tests auf der Basis des HIV-2$_{D205}$ einzusetzen, mit deren Hilfe man HIV-2$_{D205}$ von anderen HIV-Viren unterscheiden kann.

Im folgenden werden die Figuren sowie die Tabelle beschrieben:

Tabelle 1 zeigt die Nukleotidsequenzhomologie zwischen HIV und SIV-Viren in Prozent. Die Sequenzen wurden verglichen unter Benutzung von Mikrogenie™-Sequenz Software der Fa. Beckmann.

Tabelle 2 zeigt die erhaltenen Sequenzen der genetischen Elemente des Nukleotid- und Aminosäurebereichs.

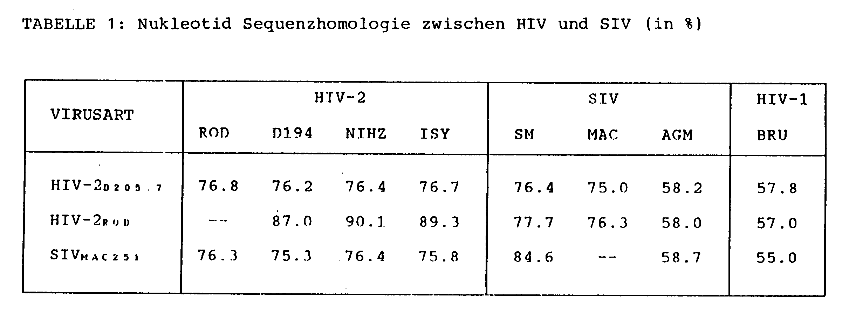

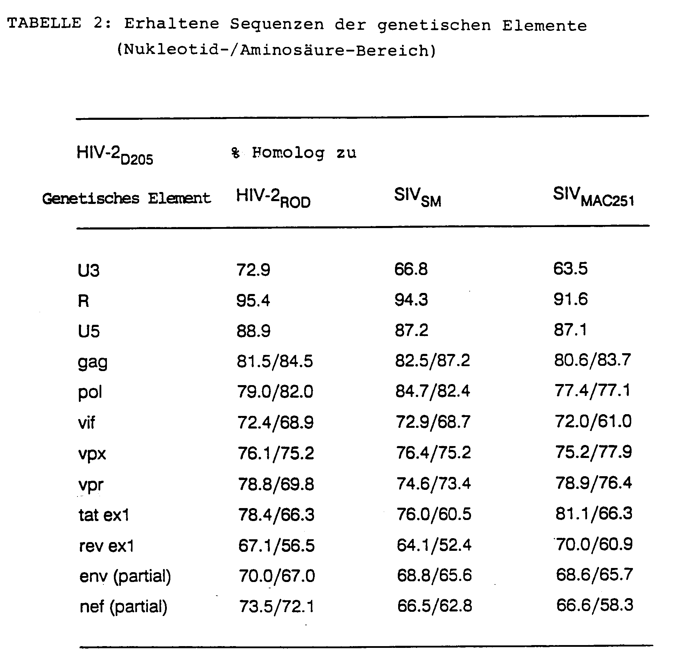

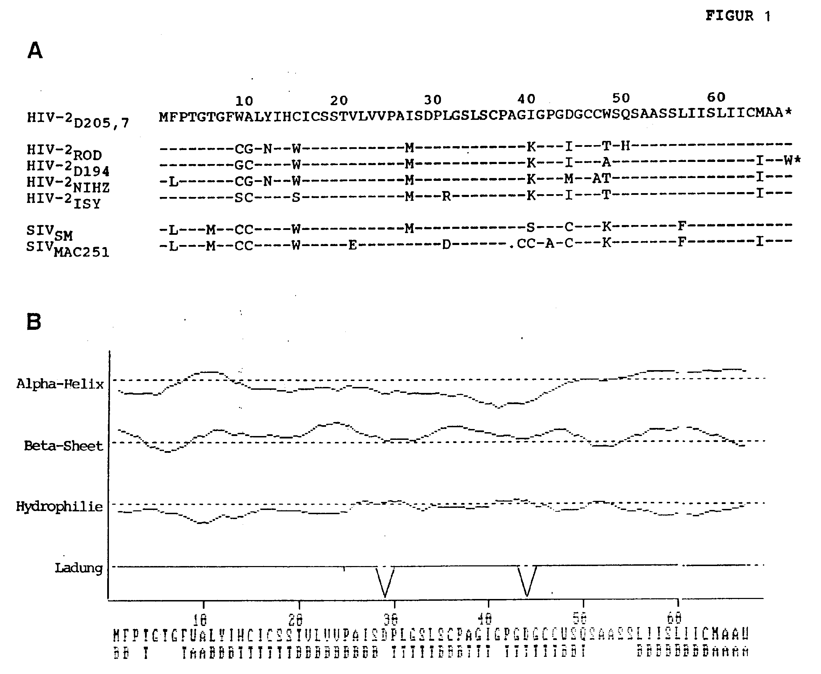

Figur 1 zeigt die Analyse der deduzierten Aminosäuresequenz des ORF-Y (open reading frame) der Positionen 1851 bis 1651 auf dem gegenstrang der HIV-2$_{D205}$-Sequenz. a) zeigt die Aminosäurenausrichtung der putativen Proteine des ORF-Y verschiedener HIV-2-Stämme, SIV$_{sm}$ und SIV$_{mac}$. b) zeigt die Profile der deduzierten Proteine des ORF-Y von HIV-2$_{D205}$ unter Benutzung des Nikrogenie™-Analyseprogramms von Beckmann.

Figur 2 zeigt die Aminosäuresequenz der externen Glycoproteine der Viren der HIV-2/SIV$_{sm}$/SIV$_{mac}$-Gruppe. Der sequenzierte Klon HIV-2$_{D205}$ enthielt nur 75 % des externen Glycoproteins. Die potentiellen N-Glyosylationsstellen sind unterstrichen. Die erhaltenen Cysteinstellen sind mit einem Sternchen versehen.

Figur 3 zeigt einen Größenvergleich der externen Glycoproteine des HIV-2$_{ROD}$ mit denen des HIV-2$_{D205}$. $^{35}$S-Cystein angereicherte Proteine von Zellen wurden mit HIV-2$_{ROD}$ (Spalte 1 und 2) und HKIV-2$_{D205}$ (Spalte 3 und 4) infiziert, mit HIV-2 positivem Serum immunopräzipitiert und mit einem 8,5 %igen SDS-Polyacrylamidgel aufgetrennt. Das externe Glycoprotein von HIV-2$_{ROD}$ (gp 125) und seine Vorstufe (gp 140), die dimere Form des gp 140 (gp 300) und die Positionen der molekularen Größenangaben sind in Dalton angegeben. Bei HIV-2$_{D205}$ infizierten Zellen ist die ein Dimer kennzeichnende Bande nicht zu erkennen. Auch nach Behandlung mit Castanospermin, Spalte 4, einem Inhibitor der Glukosidase 1, tritt die dimere Form nur bei HIV-2$_{ROD}$, Spalte 2, auf. Weiterhin bemerkenswert ist, daß das externe Glycoprotein und seine Vorstufe bei HIV-2$_{D205}$ größer sind als bei HIV-2$_{ROD}$.

Figur 4 zeigt den Evolutionsstammbaum minimaler Länge, der den Zusammenhang des HIV-2$_{D205}$ mit den anderen HIV-/ SIV-Subtypen zeigt. Der Stammbaum wurde erstellt durch einzelne Basensubstitutionen im 3'-Bereich der HIV-2$_{D205}$-Sequenz unter Benutzung von PAUP (Smith, T.F. et al., Nature 333, 573 - 575 (1988)) und der Version 3,21 des PHYLIP bootstrapping alogarythmus.

Figur 5 zeigt die Aminosäuresequenzen, abgeleitet von den einzelnen Genen der HIV-2$_{D205}$,7 bzw. HIV-2$_{ROD}$ Sequenz, diese wurden mit den Aminosäure-Consensus-Sequenzen verglichen, die aus allen bereits sequenzierten HIV-2 und SIV$_{mac}$/SIV$_{sm}$ Sequenzen abgeleitet wurden. Unter den von der Consensus-Sequenz abweichenden Aminosäuren von HIV-2$_{D205}$,7 (5 a) bzw. HIV-2$_{ROD}$ (5 b) wurde ermittelt, welcher Prozentsatz mit der HIV-2 Consensus-Sequenz identisch ist (schwarze Balken), welcher mit der SIV$_{mac}$/SIV$_{sm}$ Consensus-Sequenz identisch ist (graue Balken) und welcher Prozentsatz neuen Aminosäuren entspricht (helle Balken).

**Patentansprüche**

1. Vakzine zum Schutz vor HIV-Virusinfektionen aus dem Virus HIV-2$_{D205}$ (ECACC V 87 122 304), enthaltend mindestens eines der Peptide

```
 1. LFETSIKPCVKL              2. ESCDKHYWD
 3. RFRYCAPPG                 4. LLRCNDTNYSGF
 5. STWFGFNGTRAENRYIYWH       6. DNRTIISLN
 7. NELDRFGLAESLLE            8. PLVPTGSENLKSL
 9. PLSPRTLNAWVKL
10. EEKKFGAEVVPGFQALSEGCTPYEINQMLNCV
11. GLQKCVRMYNPTNILD         12. FQSYVDRFYKSLRAEQTD
13. QNANPDCKLVLKGL           14. NPTLEEMLTACQG
15. GGPGQKARLMAEALKE         16. ARQCRAPRRQGCWKCGK
```

oder deren Kombinationen.

2. Vakzine nach Anspruch 1, dadurch gekennzeichnet, daß sie bis zu 7 Aminosäuren lange Unterpeptide enthält.

3. Vakzine nach Anspruch 1 und/oder 2, dadurch gekennzeichnet, daß zusätzlich die abweichenden Proteinbereiche rev (aa 2-11), vif (aa 186-202) und/oder env (aa 2-33 ; 118-204 etc.) enthalten sind.

4. Verwendung des Virus HIV-2$_{D205}$ zur Herstellung einer Vakzine gemäß mindestens einem der Ansprüche 1 bis 3 zum Schutz vor HIV-Virusinfektionen.

5. Verwendung nach Anspruch 4 zur Herstellung einer Vakzine zum therapeutischen Einsatz nach der Infektion.

6. Verfahren zur Herstellung einer Vakzine gemäß mindestens einem der Ansprüche 1 bis 3 zum Schutz vor HIV-Virusinfektionen, dadurch gekennzeichnet, daß das Virus HIV-2$_{D205}$ in einen Affen implantiert, in diesem vermehrt und die gebildeten Antikörper als Impfstoff verwendet werden.

7. Verfahren nach Anspruch 6 zur Herstellung einer Vakzine zum therapeutischen Einsatz nach der Infektion.

8. Vakzine von Peptiden aus HIV-2$_{D205}$ gemäß mindestens einem der Ansprüche 1 bis 3 zur Verwendung in einem Tiermodell zur Diagnose von AIDS und zur Differenzierung zwischen pathogenen und apathogenen HIV-Infektionen, die mit den bisherigen diagnostischen Tests als HIV-2 diagnostiziert werden und nicht unterschieden werden können.

9. Vakzine nach Anspruch 8, dadurch gekennzeichnet, daß zwischen Infektionen des Typs HIV-2$_{D205}$ und HIV-2 Infektionen, die durch den Prototyp HIV-2$_{ROD}$ definiert sind, differenziert werden kann.

**Claims**

1. A vaccine for protection against HIV infections from the virus HIV-2$_{D205}$ (ECACC V 87 122 304), containing at least one of the peptides

| | | | |
|---|---|---|---|
| 1. | LFETSIKPCVKL | 2. | ESCDKHYWD |
| 3. | RFRYCAPPG | 4. | LLRCNDTNYSGF |
| 5. | STWFGFNGTRAENRYIYWH | 6. | DNRTIISLN |
| 7. | NELDRFGLAESLLE | 8. | PLVPTGSENLKSL |
| 9. | PLSPRTLNAWVKL | | |
| 10. | EEKKFGAEVVPGFQALSEGCTPYEINQMLNCV | | |
| 11. | GLQKCVRMYNPTNILD | 12. | FQSYVDRFYKSLRAEQTD |
| 13. | QNANPDCKLVLKGL | 14. | NPTLEEMLTACQG |
| 15. | GGPGQKARLMAEALKE | 16. | ARQCRAPRRQGCWKCGK |

or combinations thereof.

2. The vaccine according to claim 1, characterized in that it contains subpeptides having a length of up to 7 amino acids.

3. The vaccine according to claim 1 and/or 2, characterized in that additionally, the deviating protein regions rev (aa 2-11), vif (aa 186-202) and/or env (aa 2-33; 118-204 etc.) are contained.

4. A use of the virus HIV-2$_{D205}$ for preparing a vaccine according to at least one of claims 1 to 3 for protection against HIV infections.

5. The use according to claim 4 for preparing a vaccine for therapeutic application subsequent to infection.

6. A process for the preparation of a vaccine according to at least one of claims 1 to 3 for protection against HIV infections, characterized in that the virus HIV-2$_{D205}$ is incorporated into a monkey, multiplied therein, and the antibodies formed are used as a vaccine.

7. The process according to claim 6 for preparing a vaccine for therapeutic application subsequent to infection.

8. A vaccine of peptides from HIV-2$_{D205}$ according to at least one of claims 1 to 3 for use in an animal model for the diagnosis of AIDS and for differentiating between pathogenic and non-pathogenic HIV infections which, with previous diagnostic assays, are diagnosed as HIV-2 and cannot be distinguished.

9. The vaccine according to claim 8, characterized in that differentiation is possible between HIV-2$_{D205}$ type infections and HIV-2 infections defined by the prototype HIV-2$_{ROD}$.

**Revendications**

1. Vaccins destinés à protéger d'infections virales dues au virus HTV-2$_{D205}$ (ECACC V 87 122 304), contenant au-moins l'un des peptides

```
1.   LFETSIKPCVKL              2.   ESCDKHYWD
3.   RFRYCAPPG                 4.   LLRCNDTNYSGF
5.   STWFGFNGTRAENRYIYWH       6.   DNRTIISLN
7.   NELDRFGLAESLLE            8.   PLVPTGSENLKSL
9.   PLSPRTLNAWVKL
10.  EEKKFGAEVVPGFQALSEGCTPYEINQMLNCV
11.  GLQKCVRMYNPTNILD          12.  FQSYVDRFYKSLRAEQTD
13.  QNANPDCKLVLKGL            14.  NPTLEEMLTACQG
15.  GGPGQKARLMAEALKE          16.  ARQCRAPRRQGCWKCGK
```

ou des combinaisons de ces peptides.

**2.** Vaccins selon la revendication 1, caractérisés en ce qu'ils comprennent des sous-peptides ayant une longueur de 7 acides aminés maximum.

**3.** Vaccins selon la revendication 1 et/ou 2, caractérisés en ce qu'ils comportent en outre des domaines protéiniques rev (aa 2-11), vif (aa 186-202) et/ou env (aa 2-33; 118-204 etc.).

**4.** Utilisation du virus HIV$^{-2}_{\text{D205}}$ pour préparer un vaccin selon l'une au-moins des revendications 1 à 3 en vue d'une protection contre les infections causées par le virus HIV.

**5.** Utilisation selon la revendication 4 pour préparer un vaccin à des fins thérapeutiques après l'infection.

**6.** Procédé de préparation d'un vaccin selon l'une au-moins des revendications 1 à 3 en vue d'une protection contre les infections dues au virus HIV, caractérisé en ce que le virus HIV$^{-2}_{\text{D205}}$ est implanté dans un singe, pour qu'il s'y multiplie, les anticorps alors formés étant utilisés comme vaccin.

**7.** Procédé selon la revendication 6 pour préparer un vaccin à des fins thérapeutiques après l'infection.

**8.** Vaccins composés de peptides du HIV$^{-2}_{\text{D205}}$ selon l'une au-moins des revendications 1 à 3 pour une utilisation dans un modèle animal à des fins de diagnostic du SIDA et pour distinguer les infections pathogènes et non-pathogènes dues au HIV, qui sont actuellement diagnostiquées à l'aide des tests diagnostiques connus jusqu'ici comme des HIV-2 et qui ne peuvent être différenciées.

**9.** Vaccins selon la revendication 8, caractérisé en ce qu'il est possible de distinguer entre les infections du type HIV$^{-2}_{\text{D205}}$ et les infections HIV-2 définies par le prototype HIV$^{-2}_{\text{ROD}}$.

TABELLE 1: Nukleotid Sequenzhomologie zwischen HIV und SIV (in %)

| VIRUSART | HIV-2 | | | | SIV | | | HIV-1 |
|---|---|---|---|---|---|---|---|---|
| | ROD | D194 | NIHZ | ISY | SM | MAC | AGM | BRU |
| HIV-2$_{D205.7}$ | 76.8 | 76.2 | 76.4 | 76.7 | 76.4 | 75.0 | 58.2 | 57.8 |
| HIV-2$_{ROD}$ | --- | 87.0 | 90.1 | 89.3 | 77.7 | 76.3 | 58.0 | 57.0 |
| SIV$_{MAC251}$ | 76.3 | 75.3 | 76.4 | 75.8 | 84.6 | -- | 58.7 | 55.0 |

EP 0 495 896 B1

TABELLE 2: Erhaltene Sequenzen der genetischen Elemente
(Nukleotid-/Aminosäure-Bereich)

| HIV-2$_{D205}$<br>Genetisches Element | % Homolog zu<br>HIV-2$_{ROD}$ | SIV$_{SM}$ | SIV$_{MAC251}$ |
|---|---|---|---|
| U3 | 72.9 | 66.8 | 63.5 |
| R | 95.4 | 94.3 | 91.6 |
| U5 | 88.9 | 87.2 | 87.1 |
| gag | 81.5/84.5 | 82.5/87.2 | 80.6/83.7 |
| pol | 79.0/82.0 | 84.7/82.4 | 77.4/77.1 |
| vif | 72.4/68.9 | 72.9/68.7 | 72.0/61.0 |
| vpx | 76.1/75.2 | 76.4/75.2 | 75.2/77.9 |
| vpr | 78.8/69.8 | 74.6/73.4 | 78.9/76.4 |
| tat ex1 | 78.4/66.3 | 76.0/60.5 | 81.1/66.3 |
| rev ex1 | 67.1/56.5 | 64.1/52.4 | 70.0/60.9 |
| env (partial) | 70.0/67.0 | 68.8/65.6 | 68.6/65.7 |
| nef (partial) | 73.5/72.1 | 66.5/62.8 | 66.6/58.3 |

FIGUR 1

EP 0 495 896 B1

## A

```
                        10        20        30        40        50        60
HIV-2 D205,7    MFPTGTGFWALYIHCICSSTVLVVPAISDPLGSLSCPAGIGPGDGCCWSQSAASSLIISLIICMAA*

HIV-2 ROD      --------CG-N--W-----------M------------K---I---T-H---------------
HIV-2 D194     --------GC----W-----------M------------K---I---A--------------I--W*
HIV-2 NIHZ     -L------CG-N--W-----------M------------K---M--AT--------------I---
HIV-2 ISY      --------SC----S-----------M---R--------K---I---T--------------I---

SIV SM         -L---M--CC----W-----------M------------S---C---K-------F----------
SIV MAC251     -L---M--CC----W-----E---------D------.CC-A-C---K-------F--------I---
```

## B

```
HIV-2_ROO      MM...NQLLIAILLASACLVYCTQYVTVFYGVPTWKNATIPLFCATRNRDTWGTIQCLPDNDDYQ 62
HIV-2_D205     -AYFSSR-P--L--IGISGFV-K---------I-A-R---V--I---T------V------G--T 65
HIV-2_D194     -EPGR----V----T----I-K---------I-A-R--S------K------------------ 65
SIV_SM         -GCLG------L--V-VLEIC-V----------A--------------K------T---------S 65
SIV_MAC251     -GCLG---------L-VYGI-------------A-R---------------K------T------G--S 65

HIV-2_ROO      EITLNVTEAFDAWNNTVTEQAIEDVWHLFETSIKPCVKLTPLCVAMKCSSTE....SSTGNNTTS 123
HIV-2_D205     --R--I-------D----Q--VN---R-------------------N--K--....TNP-:-.A- 124
HIV-2_D194     -------------D-----------R------------------N-NI-....-----..--. 122
SIV_SM         -LAI---------D-----------N------------------I--R-NK--TDRWGL---AG-T 130
SIV_MAC251     -LA----S----E-----------E-----------S---IT-R-NKS-TDRWGL-....K- 126

HIV-2_ROO      KSTSTTTTTPTDQEQEIS.....EDTPCARADNCSGLGEEETINCQFNMTGLERDKKKQYNETWY 183
HIV-2_D205     ST-T-KP--TSRGLKT-N.....-TD--IKN-S-T------IMQ-N-S----R--EL---KD--- 184
HIV-2_D194     ATP-PPNI-II-...-N-........-.-IGDN--T---K--VVE-E------Q---RK--NA-- 176
SIV_SM         TTAI---A--SVA-NV-N.....-SN--IKNNS-A--EQ-PM-G-K-----N-----E------ 190
SIV_MAC251     .--TI--AA--SAPVSEKIDMVN-TSS-IAQN--T--EQ-QM-S-K-T----K---T-E------ 185

HIV-2_ROO      SKDVVCETNNST.NQTQCYMNHCNTSVITESCDKHYWDAIRFRYCAPPGYALLRCNDTNYSGFAP 247
HIV-2_D205     -E-LE-..--IRKYTSR--IRT---TI-Q---------SL---------FF-----------M- 247
HIV-2_D194     -R----DKT-G-..G-.---R------K----------MK--------F-------------E- 238
SIV_SM         -R-LI--QSANE.SESK---H------Q--------------------------S--L---- 254
SIV_MAC251     -T-L---QG---D-ESR-----------Q---------T-------------------------M- 250

HIV-2_ROO      NCSKVVASTCTRMMETQTSTWFGFNGTRAENRTYIYWHGRDNRTIISLNKYYNLSLHCKRPGNKI 312
HIV-2_D205     ---------S--------S-------------------EK---------T----I-------T 312
HIV-2_D194     K------AS---------------------------K-----------TM--------T 303
SIV_SM         ------V-S------------------------------KS----------TMR-R--E--T 319
SIV_MAC251     K-----V-S-----------------------------------------TMK-R----T 315

HIV-2_ROO      VKQIMLMSGHVFHSHYQPINKRPRQAWCWFKGKWKDAMQEVKETLAKHPYRG..TNDTRNISFA 375
HIV-2_D205     -VP-RTV--LL---..----------------N-TE-IK---R-II-----K-GAK-I-SVKLVS 375
HIV-2_D194     -VP-T----RR---.RPVY--K-G------Q-N-IE--R---Q--------G-..----GK-N-T 365
SIV_SM         -LPVTI---L----..----E--K------E-S--K-I-------V-----T-..----K-NLT 380
SIV_MAC251     -LPVTI---L----..--LTD--K------G------IK---Q-IV-----T-..--N-DK-NKT 376

HIV-2_ROO      APGKGSDPEVAYMWTNCRGEFLYCNMTWFLNWIENK...............IHRNYAPCHIKQII 425
HIV-2_D205     EH----- 382
HIV-2_D194     K--I------T-----------------------V--TN.............--G-------R--- 427
SIV_SM         --.A-G----TF------------K-N-----V-DRDEKGGRWKQQNRKEQQKK--V----R--- 429
SIV_MAC251     ---.-G----TF------------K-N-----V-DRDVTTQRPKQR.....HR---V----R--- 435

HIV-2_ROO      NTWHKVGRNVYLPPREGELSCNSTVTSIIANIDWQNNNQTNITFSAEVAELYRLELGDYKLVEIT 490
HIV-2_D205
HIV-2_D194     -------T-----------T---.-------.SDG----------------------I-V- 491
SIV_SM         -------K---------D-T------L--E---I-S-E----M-----------------I--- 494
SIV_MAC251     -------K---------D-T------L------TDG---S--M-------------------- 500

HIV-2_ROO      PIGFAPTKEKRY.SSAHGRHTR 511
HIV-2_D205
HIV-2_D194     --P----------.---PV-NK- 512
SIV_SM         ---L---SVR--TTTGAS-NK- 515
SIV_MAC251     ---L---DV---TTGGTS-NK- 521
```

**FIGUR 5 a)**

% Identität

HIV-2D205 proteine

■ HIV-2 Consensus  SIV Consensus  neue Aminosäure

**FIGUR 5 b)**

% Identität

ROD proteine

■ HIV-2 Consensus  SIV Consensus  neue Aminosäure